# EUROPÄISCHE PATENTSCHRIFT

(11) **EP 2 862 564 B1**
(45) Veröffentlichungstag und Bekanntmachung des Hinweises auf die Patenterteilung: **16.11.2016**
(21) Anmeldenummer: 14160438.9
(22) Anmeldetag: 18.03.2014
(51) Int. Cl.: A61K 8/44, A61K 8/46, A61K 8/60, A61Q 11/00

(54) **Mund- und Zahnreinigungsmittel**
Oral and dental hygiene agent
Produit de nettoyage de la bouche et des dents

(30) Priorität: 05.08.2013 DE 102013215344
(43) Veröffentlichungstag der Anmeldung: 22.04.2015
(73) Patentinhaber: Henkel AG & Co. KGaA, 40589 Düsseldorf (DE)
(72) Erfinder: Miehlich, Kristin, 42119 Wuppertal (DE); Adelmann, Barbara, 40597 Düsseldorf (DE); Welß, Thomas, 40591 Düsseldorf (DE); Döring, Thomas, 41540 Dormagen (DE)

(56) Entgegenhaltungen:
- EP-A2- 1 216 688
- EP-A2- 2 371 347
- WO-A2-2013/020960
- US-A- 5 833 956

## Beschreibung

Die vorliegende Erfindung betrifft ein Mund- und Zahnreinigungsmittel mit einem äußerst geringen Gehalt an Alkyl(ether)sulfaten, welches hervorragende Schaumeigenschaften aufweist und sich insbesondere für entzündetes, gereiztes und sensibles Zahnfleisch eignet.

Weiterhin betrifft die vorliegende Erfindung die Verwendung des erfindungsgemäßen Mund- und Zahnreinigungsmittels zur Reinigung und Pflege der Zähne und/oder der Mundhöhle.

Mund- und Zahnreinigungsmittel sind in verschiedenen Formen auf dem Markt und dienen in erster Linie der Reinigung der Zahnoberfläche, der Vorbeugung von Zahn- und Zahnfleischerkrankungen sowie der Bekämpfung von Mundgeruch.

Zahnreinigungsmittel enthalten üblicherweise eine Kombination aus Poliermitteln, Feuchthaltemitteln, Tensiden, Bindemitteln, Aromastoffen und fluoridhaltigen sowie antimikrobielle Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes zunehmend an Bedeutung gewonnen.

Zahnreinigungsmittel in Form von Zahnpasten, Zahncremes und Zahngelen werden insbesondere bei der täglichen Reinigung der Zähne durch manuell betätigte oder elektrische Zahnbürsten angewandt. Die Zahnreinigungsmittel sollen in erster Linie die Reinigung der Zahnoberfläche von Speiseresten, Verfärbungen durch z. B. Tabak oder Tee und von den fest anhaftenden bakteriellen Zahnbelägen, der sogenannten Plaque, gewährleisten. Dies geschieht einerseits durch die mechanische Wirkung der manuellen oder elektrischen Zahnbürste in Kombination mit den Poliermittelkomponenten, andererseits durch die eingesetzten Tenside, welche die mechanische Zahnbelagsentfernung, insbesondere an Stellen, welche mit der Zahnbürste nur schwer erreicht werden können, unterstützen. Die entfernten Verunreinigungen, wie Plaque- und Speisereste, werden durch die Tenside dispergiert, so dass ein erneutes Absetzen auf der Zahnoberfläche verhindert wird. Darüber hinaus sollen die eingesetzten Tenside eine gewisse Schaummenge erzeugen, um eine gleichmäßige Verteilung der Komponenten des Zahnreinigungsmittels während des Zähneputzens im Mund zu gewährleisten. Die in Zahnreinigungsmitteln eingesetzten Tenside müssen gut haut- bzw. schleimhautverträglich und toxikologisch unbedenklich sein, um eine Gefährdung der Verbraucher zu vermeiden. Schließlich muss das Zahnreinigungsmittel eine gute Schaumstabilität und Schaumkonsistenz aufweisen, da dies wichtige Faktoren für die Produktakzeptanz durch den Verbraucher darstellen.

Mund- und Zahnreinigungsmittel des Standes der Technik, insbesondere Mund- und Zahnreinigungsmittel mit hohen Gehalten an Feuchthaltemitteln, weisen oftmals eine unzureichende Schaummenge und Schaumstabilität auf. Eine zu geringe Schaumstabilität führt zu einer Verflüssigung des Schaums und lässt diesen vorzeitig aus dem Mund tropfen, was vom Verbraucher als unangenehm empfunden wird. Dies führt dazu, dass eine nur ungenügende Reinigung der Zähne durch den Verbraucher erfolgt, so dass vermehrt Zahnfleischerkrankungen, welche als "Parodontitis" oder auch "entzündliche Parodontopathie" bezeichnet werden, auftreten. Aus diesem Grund wurde im Stand der Technik versucht, die Schaumstabilität sowie die Schaumenge der Mund- und Zahnreinigungsmittel durch Erhöhung der Menge an schaumstarken anionischen Tensiden zu verbessern. Jedoch resultiert eine Erhöhung der Konzentration dieser Tenside in Zahnfleisch- und Schleimhautreizungen, so dass sich diese Mund- und Zahnreinigungsmittel nicht für Verbraucher eignen, welche aufgrund von Zahnfleischerkrankungen bereits unter entzündetem, gereiztem oder sensiblem Zahnfleisch leiden, da hohe Konzentrationen an schleimhautreizenden Tensiden zu einer Verschlimmerung der Symptome führen.

Es besteht daher ein Bedarf an Mund- und Zahnreinigungsmitteln, welche nur geringe Mengen an hautreizenden anionischen Tensiden enthalten, gleichzeitig jedoch eine hervorragende Schaummenge, Schaumstabilität und Schaumqualität aufweisen.

Diese Problemstellung und ähnliche Tensid-Systeme wurden im Stand der Technik in z.B. US5833956 schon beschrieben.

Der vorliegenden Erfindung lag die Aufgabe zugrunde, Mund- und Zahnreinigungsmittel bereitzustellen, welche eine hohe Schaummenge, Schaumstabilität und Schaumqualität aufweisen und sich für die Verwendung bei entzündetem, gereiztem und sensiblem Zahnfleisch eignen.

Es wurde nun gefunden, dass sich Mund- und Zahnreinigungsmittel formulieren lassen, welche sich aufgrund der geringen Menge an anionischen hautreizenden Tensiden für entzündetes, gereiztes und sensibles Zahnfleisch eignen und gleichzeitig eine hohe Schaummenge, Schaumstabilität und Schaumqualität aufweisen, wenn mindestens ein anionisches, mindestens ein amphoteres und mindestens ein nichtionisches Tensid eingesetzt werden. Die Mund- und Zahnreinigungsmittel eignen sich darüber hinaus auch zur Behandlung von Gingivits oder Parodontitis.

Gegenstand der vorliegenden Erfindung ist somit ein Mund- und Zahnreinigungsmittel, enthaltend
a) 0,1 bis 3 Gew.-%, bezogen auf das Gesamtgewicht des Mund- und Zahnreinigungs-mittels, mindestens eines Alkyl(ether)sulfats (A) der allgemeinen Formel (I)

   R¹-(O-CH₂-CH₂)ₓ-O-SO₃- X⁺ (I),

   in der R¹ für eine lineare oder verzweigte, gesättigte oder ungesättigte Alkylgruppe mit 8 bis 30 Kohlenstoffatomen, x für die Zahl 0 oder für Zahlen von 1 bis 12 und X⁺ für ein Alkalimetall- oder ein Ammoniumion steht,
b) 0,1 bis 4 Gew.-%, bezogen auf das Gesamtgewicht des Mund- und Zahnreinigungs-mittels, mindestens eines amphoteren Tensids (B) der allgemeinen Formel (II) 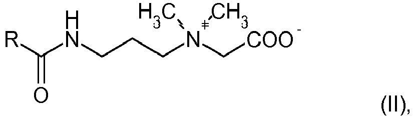 in der R für eine lineare oder verzweigte, gesättigte oder ein- oder mehrfach ungesättigte Alkyl- oder Alkenylgruppe mit 8 bis 24 Kohlenstoffatomen steht,
c) 0,1 bis 2 Gew.-%, bezogen auf das Gesamtgewicht des Mund- und Zahnreinigungs-mittels, mindestens eines nichtionischen Tensids (C) der allgemeinen Formel (III)

   R²O-[G]ₚ (III),

   in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, und
d) 0,1 bis 1 Gew.-%, bezogen auf das Gesamtgewicht des Mund- und Zahnreinigungs-mittels, mindestens eines Wirkstoffs für die orale Prophylaxe (D), ausgewählt aus der Gruppe der Fluoride, der antibakteriellen Wirkstoffe und der Phosphate, wobei das Gewichtsverhältnis der Tenside (A) : (B): (C) 0,7 : 1,8 : 1,8 bis 1 : 3,5 : 0,5 beträgt.

Die Mund- und Zahnreinigungsmittel der vorliegenden Erfindung weisen nur geringe Konzentrationen an Alkyl(ether)sulfaten, wie beispielsweise Natriumlaurylsulfat, auf, welche die natürliche Balance der Feuchtigkeit auf der Haut negativ beeinflussen und schon bei Konzentrationen von etwa 3 Gew.-% zu Schleimhautbrennen und Zellschäden führen können. Trotz der äußerst geringen Menge an Alkyl(ether)sulfaten in den erfindungsgemäßen Mund- und Zahnreinigungsmitteln wird durch die gezielte Kombination mit mindestens einem amphoteren und mindestens einem nichtionischen Tensid eine vergleichbare bzw. sogar eine höhere Schaummenge erreicht, wie bei Einsatz von mehr als doppelt so hohen Mengen an Alkyl(ether)sulfaten. Die Schaummenge und die Schaumstabilität der erfindungsgemäßen Mund-und Zahnreinigungsmittel kann durch den Einsatz der vorgenannten Tensidkombination in ganz speziellen Gewichtsverhältnissen und Mengen sogar noch weiter gesteigert werden. Die hohe Schaummenge und Schaumstabilität der erfindungsgemäßen Mund- und Zahnreinigungsmittel sorgt insbesondere bei entzündetem, gereiztem und sensiblem Zahnfleisch für ein "Kissen" zwischen Zahnbürste und Zahnfleisch und führt somit zu einer schonenderen, schmerzfreieren und gründlicheren Reinigung der Zähne. Weiterhin können bei Verwendung der vorgenannten Tensidkombination, insbesondere in Kombination mit ganz speziellen Gewichtsverhältnissen und Mengen, selbst hohe Mengen an Feuchthaltemitteln eingesetzt werden, ohne das die Schaummenge und Schaumstabilität negativ beeinflusst wird.

Zudem weisen die erfindungsgemäßen Mund- und Zahnreinigungsmittel eine hohe Schaumstabilität und eine hohe Schaumqualität auf, so dass sich der Schaum cremig und angenehm anfühlt und während des Putzvorgangs nicht übermäßig aus dem Mund fließt.

Mund- und Zahnreinigungsmittel im Sinne der vorliegenden Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnreinigungsmittel z.B. in Form von Zahnpasten, flüssigen Zahncremes, Zahnpulvern, Mundwässern, Mundspülungen oder gegebenenfalls auch als Kaumasse, z. B. als Kaugummi, vorliegen. Bevorzugt liegen sie jedoch als mehr oder weniger fließfähige oder plastische Zahnpasten, wie sie zur Reinigung der Zähne unter Einsatz einer Zahnbürste verwendet werden, oder als Mundwässer bzw. Mundspülungen vor.

Unter Wirkstoffen für die orale Prophylaxe (D) werden im Sinne der vorliegenden Erfindung Wirkstoffe verstanden, welche die Entstehung von Plaque, Zahnstein, Zahnfleischentzündungen und Karies verhindern bzw. zumindest abschwächen sollen oder welche die Empfindlichkeit gegenüber Wärme, Kälte, Süßem und Saurem vermindern sollen. Hierunter fallen insbesondere Fluoride, welche den Zahnschmelz härten und auf diese Weise Karies verhindern. Weiterhin werden hierunter auch antibakterielle Wirkstoffe, Phosphate und Zinkverbindungen verstanden, welche Zahnfleischentzündungen sowie Zahnstein reduzieren bzw. die Entstehung verhindern. Zudem werden hierunter auch Kaliumverbindungen sowie Aminofluoride verstanden, welche empfindliche Zahnhälse verschließen und dadurch eine erhöhte Empfindlichkeit gegenüber Wärme, Kälte, Süßem und Saurem vermindern.

Gemäß einer bevorzugten Ausführungsform der vorliegenden Erfindung ist das mindestens eine Alkyl(ether)sulfat (A) der allgemeinen Formel (I) ausgewählt aus der Gruppe der geradkettigen oder verzweigten Alkyl(ether)sulfate mit einem Alkylrest mit 8 bis 18, insbesondere mit 10 bis 16 Kohlenstoffatomen und 0 oder 1 bis 6 Ethylenoxideinheiten, bevorzugt Natriumlaurylsulfat und/oder Natriumlaurethsulfat. Besonders gute Ergebnisse in Bezug auf die Schaummenge und die Schaumstabilität werden im Rahmen der vorliegenden Erfindung erhalten, wenn als anionisches Tensid Natriumlaurylsulfat in Kombination mit mindestens einem amphoteren und mindestens einem nichtionischen Tensid eingesetzt wird.

In diesem Zusammenhang ist es besonders bevorzugt, wenn das mindestens eine Alkyl(ether)sulfat (A) der allgemeinen Formel (I) in einer Menge von 0,1 bis 2,5 Gew.-%, insbesondere 0,3 bis 2 Gew.-%, vorzugsweise 0,4 bis 1,5 Gew.-%, bevorzugt 0,5 bis 1,3 Gew.-%, besonders bevorzugt 0,6 bis 1 Gew.-%, am meisten bevorzugt 0,7 bis 0,9 Gew.-%, bezogen auf das Gesamtgewicht des Mund- und Zahnreinigungsmittels, enthalten ist. Die vorstehend genannten äußerst geringen Mengen der hautreizenden Alkyl(ether)sulfate führen zu einer deutlich verbesserten Hautverträglichkeit, insbesondere Schleimhautverträglichkeit. Aufgrund der hervorragenden Haut- bzw. Schleimhautverträglichkeit der erfindungsgemäßen Mund- und Zahnreinigungsmittel können diese selbst bei entzündetem, gereiztem oder sensiblem Zahnfleisch angewendet werden, ohne dass die Gefahr einer Verschlechterung bereits vorhandener Symptome besteht.

Gemäß einer weiteren bevorzugten Ausführungsform der vorliegenden Erfindung ist das mindestens eine amphotere Tensid (B) der allgemeinen Formel (II) ausgewählt aus der Gruppe der C₈₋₁₈-Alkylamido(C₁₋₄)-alkylbetaine, insbesondere C₈₋₁₂-Alkylamido(C₁₋₄)-alkylbetaine.

Besonders gute Ergebnisse werden im Rahmen der vorliegenden Erfindung erhalten, wenn das mindestens eine amphotere Tensid (B) der allgemeinen Formel (II) in einer Menge von 0,1 bis 3,7 Gew.-%, insbesondere 0,5 bis 3,5 Gew.-%, vorzugsweise 1 bis 3,3 Gew.-%, bevorzugt 1,5 bis 3 Gew.-%, besonders bevorzugt 2,2 bis 2,8 Gew.-%, bezogen auf das Gesamtgewicht des Mund-und Zahnreinigungsmittels, enthalten ist. Durch den Einsatz von geringen Mengen an besonders hautverträglichen amphoteren Tensiden kann die Menge an hautreizenden Alkyl(ether)sulfaten signifikant reduziert werden, ohne jedoch die Schaummenge und die Schaumqualität negativ zu beeinflussen.

Erfindungsgemäß ist das mindestens eine nichtionische Tensid (C) der allgemeinen Formel (III) ausgewählt aus der Gruppe der Alkyloligoglucoside mit 8 bis 16 Kohlenstoffatomen in der Alkylgruppe und deren Mischungen.

In diesem Zusammenhang ist es besonders bevorzugt, wenn das mindestens eine nichtionische Tensid (C) der allgemeinen Formel (III) in einer Menge von 0,1 bis 1,5 Gew.-%, insbesondere 0,1 bis 1 Gew.-%, vorzugsweise 0,1 bis 0,8 Gew.-%, bevorzugt 0,2 bis 0,6 Gew.-%, mehr bevorzugt 0,3 bis 0,5 Gew.-%, bezogen auf das Gesamtgewicht des Mund- und Zahnreinigungsmittels, enthalten ist. Durch die Verwendung der vorstehend genannten Mengen an nichtionischem Tensid kann die Menge an Alkyl(ether)sulfaten weitergehend reduziert werden, ohne dass die Schaummenge und die Schaumstabilität sowie die Schaumqualität, insbesondere die Feinporigkeit und Cremigkeit, negativ beeinflusst werden. Aufgrund der äußerst geringen Mengen an hautreizenden Alkyl(ether)sulfaten resultiert somit ein gegenüber dem Stand der Technik hautverträglicheres, insbesondere schleimhautverträglicheres, Mund- und Zahnreinigungsmittel.

Gemäß einer bevorzugten Ausführungsform der vorliegenden Erfindung beträgt das Gewichtsverhältnis der Tenside (A) : (B) 0,7 : 1,8 bis 1 : 3,5. Das Gewichtsverhältnis der Tenside (A) : (B) bezieht sich hierbei auf das Gewichtsverhältnis der Gesamtmenge an Tensid(en) (A) zu der Gesamtmenge an Tensid(en) (B).

Das Gewichtsverhältnis der Tenside (A) : (B) : (C) bezieht sich hierbei auf das Gewichtsverhältnis der Gesamtmenge an Tensid(en) (A) zu der Gesamtmenge an Tensid(en) (B) zu der Gesamtmenge an Tensid(en) (C). Überraschenderweise resultieren die vorgenannten Gewichtsverhältnisse der Tenside (A), (B) und (C) in einer besonders hohen Schaummenge sowie in einem besonders stabilen und feinporigen Schaum. Zudem erlaubt der Einsatz einer spezifischen Menge an Tensid (B) und (C) eine signifikante Reduzierung der Menge des hautreizenden Tensids (A), so dass erfindungsgemäß ein äußerst hautverträgliches, insbesondere schleimhautverträgliches, Mund- und Zahnreinigungsmittel resultiert. Darüber hinaus wird bei Einsatz der zuvor genannten Gewichtsverhältnisse der Tenside aufgrund der hohen Schaummenge eine hervorragende Reinigungsleistung der erfindungsgemäßen Mund- und Zahnreinigungsmittel gewährleistet.

Besonders gute Ergebnisse im Hinblick auf die Schaummenge und die Schaumstabilität werden für Mund- und Zahnreinigungsmittel erhalten, bei welchen das Gewichtsverhältnis der Tenside (A) : (B) : (C) (1-1,1) : (1,4-1,8) : (1,4-1,8) beträgt. Das Gewichtsverhältnis der Tenside (A) : (B) : (C) bezieht sich hierbei auf das Gewichtsverhältnis der Gesamtmenge an Tensid(en) (A) zu der Gesamtmenge an Tensid(en) (B) zu der Gesamtmenge an Tensid(en) (C). Überraschenderweise kann die Schaummenge sowie die Schaumstabilität noch weiter gesteigert werden, wenn das Gewichtsverhältnis der Tenside (A) : (B) : (C) (1-1,1) : (1,4-1,8) : (1,4-1,8) beträgt und das Tensid (A) in einer Menge von 0,75 bis 0,95 Gew.-%, bezogen auf das Gesamtgewicht des Mund- und Zahnreinigungsmittels, und die Tenside (B) und (C) jeweils in einer Menge von 1,1 bis 1,3 Gew.-%, bezogen auf das Gesamtgewicht des Mund- und Zahnreinigungsmittels, enthalten sind.

Ebenfalls besonders gute Ergebnisse im Hinblick auf die Schaummenge und die Schaumstabilität werden für Mund- und Zahnreinigungsmittel erhalten, bei welchen das Gewichtsverhältnis der Tenside (A) : (B) : (C) (1-1,3) : (3-3,8) : (0,5-0,6) beträgt. Das Gewichtsverhältnis der Tenside (A) : (B) : (C) bezieht sich hierbei auf das Gewichtsverhältnis der Gesamtmenge an Tensid(en) (A) zu der Gesamtmenge an Tensid(en) (B) zu der Gesamtmenge an Tensid(en) (C). Überraschenderweise kann die Schaummenge sowie die Schaumstabilität auch in diesem Fall noch weiter gesteigert werden, wenn das Gewichtsverhältnis der Tenside (A) : (B) : (C) (1-1,3) : (3-3,8) : (0,5-0,6) beträgt und das Tensid (A) in einer Menge von 0,75 bis 0,95 Gew.-%, bezogen auf das Gesamtgewicht des Mund- und Zahnreinigungsmittels, und das Tensid (B) in einer Menge von 2,3 bis 2,9 Gew.-%, bezogen auf das Gesamtgewicht des Mund- und Zahnreinigungsmittels, und das Tensid (C) in einer Menge von 0,4 bis 0,5 Gew.-%, bezogen auf das Gesamtgewicht des Mund- und Zahnreinigungsmittels, enthalten ist.

Die Kombination der vorstehend genannten Gewichtsverhältnisse der drei Tenside (A), (B) und (C) mit den vorstehend genannten Mengenbereichen der Tenside resultiert überraschenderweise in einer weiteren Erhöhung der Schaummenge gegenüber dem Stand der Technik. Weiterhin weist der Schaum eine hervorragende Stabilität auf und ist besonders feinporig und cremig. Durch die selektive Kombination der vorstehend genannten Tenside (A), (B) und (C) in speziellen Gewichtsverhältnissen und Mengenbereichen ist es der Anmelderin in überraschender Weise gelungen, die Mengen an Alkyl(ether)sulfaten gegenüber Mund- und Zahnreinigungsmitteln des Standes der Technik signifikant zu reduzieren, ohne jedoch die Schaummenge und die Schaumstabilität der erfindungsgemäßen Mund- und Zahnreinigungsmittel negativ zu beeinflussen. Aufgrund der äußerst geringen Menge an hautreizenden Alkyl(ether)sulfaten sind die erfindungsgemäßen Mund- und Zahnreinigungsmittel insbesondere für entzündetes, gereiztes und sensibles Zahnfleisch geeignet.

Die erfindungsgemäßen Mund- und Zahnreinigungsmittel enthalten weiterhin mindestens einen Wirkstoff für die orale Prophylaxe (D), wobei der mindestens eine Wirkstoff für die orale Prophylaxe (D) bevorzugt ausgewählt ist aus der Gruppe der (i) Natriumfluoride; (ii) Dinatriummonofluorphosphate; (iii) Kaliumfluoride; (iv) Zinn(II)-fluoride; (v) Aminfluoride sowie (vi) deren Mischungen, insbesondere Natriumfluorid. Der Einsatz von Fluoriden in den erfindungsgemäßen Mund- und Zahnreinigungsmitteln führt einerseits zu einer Härtung des Zahnschmelzes und beugt Karieserkrankungen in effektiver Weise vor. Anderseits führt die Verwendung von Aminfluoriden zur Schließung von Dentinkanälen bei freiliegenden Zahnhälsen, so dass bei Einsatz der erfindungsgemäßen Mund- und Zahnreinigungsmittel die Empfindlichkeit gegenüber Warmem, Kaltem, Süßem und Sauren verringert werden kann.

Gemäß einer weiteren Ausführungsform der vorliegenden Erfindung kann der mindestens eine Wirkstoff für die orale Prophylaxe (D) ausgewählt sein aus Cetylpyridiniumchlorid, Chlorhexidin, Hexetidin, Domiphenchlorid, Triclosan, Benzoaten sowie deren Mischungen, insbesondere Cetylpyridiniumchlorid. Die vorstehend genannten antimikrobiell wirkenden Verbindungen eignen sich insbesondere zur Vermeidung bzw. Hemmung der Plaquebildung sowie zur Vermeidung oder Behandlung von Zahnfleischentzündungen, welche durch Bakterien verursacht werden.

In diesem Zusammenhang kann das erfindungsgemäße Mund- und Zahnreinigungsmittel weiterhin mindestens einen Wirkstoff für die orale Prophylaxe (D), ausgewählt aus der Gruppe der (i) Ethylendiamintetraessigsäure und deren Natriumsalzen; (ii) Pyrophosphatsalze, insbesondere wasserlösliche Dialkali- oder Tetraalkalimetallpyrophosphatsalze; (iii) Polyphosphatsalze, insbesondere Alkalimetalltripolyphosphate, Natriumtripolyphosphate und Kaliumtripolyphosphate; sowie (iv) deren Mischungen enthalten. Die wasserlöslichen Dialkali- und Tetraalkalimetallpyrophosphate können insbesondere ausgewählt sein aus Na₄P₂O₇, K₄P₂O₇, Na₂K₂P₂O₇, Na₂H₂P₂O₇ sowie K₂H₂P₂O₇. Diese Substanzen wirken als Chelatbildner und vermeiden die Entstehung bzw. erleichtern die Entfernung von Zahnstein, welcher durch die Einlagerung von anorganischen Substanzen aus dem Speichel in die Plaque gebildet wird und die Entstehung von Paradontitis begünstigt.

Erfindungsgemäß kann der mindestens eine Wirkstoff für die orale Prophylaxe (D) in einer Menge von 0,1 bis 0,8 Gew.-%, insbesondere 0,1 bis 0,7 Gew.-%, vorzugsweise 0,1 bis 0,6 Gew.-%, bevorzugt 0,1 bis 0,5 Gew.-%, besonders bevorzugt 0,1 bis 0,3 Gew.-%, bezogen auf das Gesamtgewicht des Mund- und Zahnreinigungsmittels, enthalten sein. Diese Mengen gewährleisten eine effektive Prophylaxe gegen die Bildung von Karies, Zahnstein und Plaque und beugen so der Entstehung von Zahn- und Zahnfleischerkrankungen, wie Paradontitis vor.

Die erfindungsgemäßen Mund- und Zahnreinigungsmittel können zusätzlich teilchenförmige Polierkomponenten, ausgewählt aus der Gruppe der Fällungs- und Gelkieselsäuren, Aluminiumhydroxide, Aluminiumoxide, Natriumaluminiumsilikate, organischen Polymere, Hydroxylapatite, Natriumaluminiumsilicate sowie deren Mischungen, insbesondere Fällungs- und Gelkieselsäuren, enthalten, wobei die teilchenförmigen Polierkomponenten einen mittleren Teilchendurchmesser D50 von kleiner 15 µm aufweisen und/oder wobei die teilchenförmigen Polierkomponenten in einer Menge von 1 bis 40 Gew.-%, insbesondere 2 bis 35 Gew.-%, vorzugsweise 3 bis 30 Gew.-%, bevorzugt 4 bis 25 Gew.-%, besonders bevorzugt 5 bis 20 Gew.-%, bezogen auf das Gesamtgewicht des Mund- und Zahnreinigungsmittels, enthalten sind. Die vorstehend genannten Mengen der eingesetzten Polierkomponenten gewährleisten eine effektive mechanische Entfernung von Speiseresten und der Plaque und beugen so Zahn- und Zahnfleischerkrankungen vor. Besonders bevorzugt werden Polierkomponenten eingesetzt, welche einen Teilchendurchmesser von kleiner 15 µm aufweisen, um ein sandiges und unangenehmes Gefühl des Mund- und Zahnreinigungsmittels zu vermeiden und angenehme organoleptische Eigenschaften zu gewährleisten.

Die Bestimmung des mittleren Teilchendurchmessers D50 der Polierkomponenten kann beispielsweise mittels dynamischer Lichtstreuung gemäß der DIN ISO 22412 erfolgen.

Erfindungsgemäß besonders bevorzugt werden Polierkomponenten eingesetzt, welche keine Calciumionen enthalten. Es ist im Rahmen der vorliegenden Erfindung jedoch auch möglich, bis zu 5 Gew.-%, bezogen auf das Gesamtgewicht des Mund- und Zahnreinigungsmittels, an calciumhaltigen Polierkomponenten, wie Kreide, Calciumpyrophosphat oder Dicalciumphosphatdihydrat sowie deren Mischungen, einzusetzen.

Gemäß einer weiteren bevorzugten Ausführungsform der vorliegenden Erfindung werden als Polierkomponenten Kieselsäuren, wie Gelkieselsäuren, Hydrogelkieselsäuren und Fällungskieselsäuren, eingesetzt.

Gelkieselsäuren werden durch Umsetzung von Natriumsilikatlösungen mit starken, wässrigen Mineralsäuren unter Ausbildung eines Hydrosols, Alterung zum Hydrogel und anschließendem Waschen und Trocknen hergestellt. Erfolgt die Trocknung unter schonenden Bedingungen auf Wassergehalte von 15 bis 35 Gew.-%, werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Fällungskieselsäuren werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Bevorzugt werden Fällungskieselsäuren mit einer BET-Oberfläche von 15 bis 110 m²/g, einer Partikelgröße von 0,5 bis 20 µm, wobei wenigstens 80 Gew.-% der Primärpartikel unter 5 µm liegen sollen, und einer Viskosität in 30 %-tiger Glycerin-Wasser-(1 : 1)-Dispersion von 30 bis 60 Pa*s (20°C) in einer Menge von 10 bis 20 Gew.-% des erfindungsgemäßen Mund- und Zahnreinigungsmittels eingesetzt. Bevorzugt geeignete Fällungskieselsäuren dieser Art weisen außerdem gerundete Ecken und Kanten auf und sind beispielsweise unter der Handelsbezeichnung Sident^{®}12 DS (DEGUSSA) erhältlich.

Weitere erfindungsgemäß geeignete Fällungskieselsäuren sind beispielsweise Sident^{®} 8 (Evonik) und Sorbosil^{®} AC 39 (PQ Corporation). Diese Kieselsäuren zeichnen sich durch eine geringere Verdickungswirkung und eine etwas höhere mittlere Teilchengröße von 8 bis 14 µm bei einer spezifischen Oberfläche von 40 bis 75 m²/g (nach BET) aus und eignen sich besonders gut für flüssige Zahnreinigungsmittel mit einer Viskosität (25°C, Scherrate D = 10 s⁻¹) von 10 bis 100 Pa*s. Zahnreinigungsmittel, welche eine Viskosität von mehr als 100 Pa*s (25° C, Scherrate D = 10 s⁻¹) aufweisen, enthalten bevorzugt neben den zuvor genannten Fällungskieselsäuren einen ausreichend hohen Anteil an sogenannten Verdickungs-Kieselsäuren mit einer Teilchengröße von weniger als 5 µm, bevorzugt wenigstens 3 Gew.-% einer Kieselsäure mit einer Partikelgröße von 1 bis 3 µm und einer BET-Oberfläche von 150 bis 250 m²/g, wie sie beispielsweise unter dem Handelsnamen Sident^{®} 22S (DEGUSSA) erhältlich sind.

Als Polierkomponente kann erfindungsgemäß auch Aluminiumoxid in Form von schwach calcinierter Tonerde in einer Menge von 1 bis 5 Gew.-% enthalten sein. Ein geeignetes Aluminiumoxid ist beispielsweise unter der Handelsbezeichnung "Poliertonerde P10 feinst" (Giulini Chemie) erhältlich.

Zudem eigenen sich als Polierkomponenten im Rahmen der vorliegenden Erfindung weitere für den Einsatz in Zahnreinigungsmittel bekannte Polierkomponenten, wie Natriumaluminiumsilikate z. B. in Form von Zeolith A, organische Polymere wie z. B. Polymethacrylat, oder deren Mischungen. Darüber hinaus können diese Polierkomponenten auch als Mischung mit den vorstehend genannten Polierkomponenten enthalten sein.

Gemäß einer bevorzugten Ausführungsform der vorliegenden Erfindung enthält das erfindungsgemäße Mund- und Zahnreinigungsmittel mindestens einen weiteren Inhaltsstoff, ausgewählt aus der Gruppe der Lösungsvermittler, Bindemittel, Feuchthaltemittel und Konsistenzgeber, Aromaöle, Süßstoffe, Konservierungsmittel, Lösungsmittel, Farbstoffe, pH-Stellmittel und Puffersubstanzen.

Zur Solubilisierung von wasserunlöslichen Substanzen, wie Aromaölen, können die erfindungsgemäßen Mund- und Zahnreinigungsmittel einen Lösungsvermittler in einer Menge von 0,1 bis 5 Gew.-%, insbesondere 0,1 bis 3 Gew.-%, vorzugsweise 0,1 bis 1 Gew.-%, bezogen auf das Gesamtgewicht des Mund- und Zahnreinigungsmittels, enthalten. Erfindungsgemäß bevorzugt werden nichtionogene Lösungsvermittler eingesetzt, welche ausgewählt sind aus der Gruppe der oxethylierten Fettsäureglyceride, der oxethylierten Fettsäuresorbitanpartialester, der Fettsäurepartialester von Glycerin- oder Sorbitan-Oxethylaten und der Anlagerungsprodukte von 20 bis 60 Mol Ethylenoxid an gehärtetes oder ungehärtetes Ricinusöl, an Glycerinmono- und/oder - distearat und an Sorbitanmono- und/oder -distearat.

Im Rahmen der vorliegenden Erfindung können als oxethylierte Fettsäureglyceride Anlagerungsprodukte von etwa 20 bis 60 Mol Ethylenoxid an Mono- und Diglyceride von linearen Fettsäuren mit 12 bis 18 Kohlenstoffatomen oder an Triglyceride von Hydroxyfettsäuren wie Oxystearinsäure oder Ricinolsäure, eingesetzt werden. Weitere im Rahmen der vorliegenden Erfindung geeignete Lösungsvermittler sind Anlagerungsprodukte von 20 bis 60 Mol Ethylenoxid an Sorbitanmonoester und Sorbitandiester von Fettsäuren mit 12 bis 18 Kohlenstoffatomen, Mono-und Diester von C₁₂₋₁₈-Fettsäuren sowie Anlagerungsprodukte von 20 bis 60 Mol Ethylenoxid an 1 Mol Glycerin oder 1 Mol Sorbit.

Bevorzugt werden erfindungsgemäß Anlagerungsprodukte von 20 bis 60 Mol Ethylenoxid an gehärtetes oder ungehärtetes Ricinusöl, an Glycerinmono- und/oder -distearat und an Sorbitanmono- und/oder -distearat als Lösungsvermittler eingesetzt.

Die erfindungsgemäßen Mund- und Zahnreinigungsmittel können weiterhin ein Bindemittel in einer Menge von 0,1 bis 5 Gew.-%, insbesondere 0,1 bis 3 Gew.-%, vorzugsweise 0,2 bis 1 Gew.-%, bezogen auf das Gesamtgewicht des Mund- und Zahnreinigungsmittels, enthalten. Die erfindungsgemäß eingesetzten Bindemittel dienen zur Einstellung der gewünschten Konsistenz sowie des Fließverhaltens der Mund- und Zahnreinigungsmittel. Bevorzugt werden Hydrokolloide aus natürlichen Quellen, wie Xanthan, Alginate, Carrageenane, Guar Gum, Carubin, Tragant sowie synthetische Bindemittel, wie Cellulose-Derivate, eingesetzt.

Die im Rahmen der vorliegenden Erfindung verwendeten Feuchthaltemittel verhindern das Austrocknen der erfindungsgemäßen Zahnreinigungsmittel. Bei Flüssigzahnreinigungsmitteln, wie Flüssigzahncremes mit fließfähiger Rheologie dienen Feuchthaltemittel als Matrix bzw. als Konsistenzgeber und werden folglich in höheren Mengen eingesetzt. Im Falle der erfindungsgemäßen Mundreinigungsmittel, wie Mundwässern und Mundspülungen, dienen Feuchthaltemittel als zusätzliche Süßungsmittel.

Wenn das erfindungsgemäße Mund- und Reinigungsmittel als Zahnreinigungsmittel konfektioniert ist, können die Feuchthaltemittel in Mengen von 1 bis 70 Gew.-%, insbesondere 5 bis 65 Gew.-%, vorzugsweise 10 bis 60 Gew.-%, bevorzugt 15 bis 55 Gew.-%, besonders bevorzugt 20 bis 50 Gew.-%, bezogen auf das Gesamtgewicht des erfindungsgemäßen Mund- und Zahnreinigungsmittel, enthalten sein. Der negative Einfluss der hohen Gehalte an Feuchthaltemitteln, welcher zu einer raschen Verflüssigung bei Zahnreinigungsmitteln des Standes der Technik führt, kann durch die erfindungsgemäße Kombination der vorstehend genannten Tenside, insbesondere in ganz speziellen Gewichtsverhältnissen und Mengen, stark vermindert bzw. gänzlich vermieden werden. Durch den Einsatz der vorstehend genannten Tenside, insbesondere in ganz speziellen Gewichtsverhältnissen und Mengen, kann somit die Schaummenge sowie die Schaumstabilität der erfindungsgemäßen Zahnreinigungsmittel trotz der hohen Gehalte an Feuchthaltemitteln gegenüber dem Stand der Technik weitergehend gesteigert bzw. verbessert werden. Somit können in den erfindungsgemäßen Zahnreinigungsmitteln wesentlich höhere Gehalte an Feuchthaltemitteln eingesetzt werden, wodurch die gewünschte Konsistenz ohne negative Einflüsse auf die Schaumenge und Schaumstabilität über einen breiten Bereich einstellbar ist.

Ist hingegen das erfindungsgemäße Mund- und Reinigungsmittel als Mundwasser bzw. Mundspülung konfektioniert, können die Feuchthaltemittel in Mengen von 0,1 bis 9 Gew.-%, insbesondere 0,2 bis 8 Gew.-%, vorzugsweise 0,3 bis 6 Gew.-%, bevorzugt 0,4 bis 5 Gew.-%, besonders bevorzugt 0,6 bis 4 Gew.-%, bezogen auf das Gesamtgewicht des erfindungsgemäßen Mund- und Zahnreinigungsmittel, eingesetzt sein.

Gemäß einer bevorzugten Ausführungsform der vorliegenden Erfindung können die Feuchthaltemittel ausgewählt sein aus der Gruppe der mehrwertigen Alkohole bzw. Polyhydroxyverbindungen, insbesondere Sorbit, Glycerin, 1,2-Propyleglycol sowie deren Mischungen. Weiterhin eignen sich auch andere mehrwertige Alkohole, insbesondere solche mit mindestens 2 OH-Gruppen, wie Mannit, Xylit, Polyethylenglycol, Polypropylenglycol und deren Mischungen. Bevorzugt sind von den vorstehend genannten Verbindungen diejenigen mit 2 bis 12 OH-Gruppen, insbesondere diejenigen mit 2, 3, 4, 5, 6 oder 10 OH-Gruppen, enthalten.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2 bis 20. Auch 1,3-Diole wie H-(CH₂)ₙ-CH(OH) CH₂CH₂OH mit n = 1 bis 20 sind im Rahmen der vorliegenden Erfindung einsetzbar. Die (n,n+1)- bzw. (n,n+2)-Diole mit nicht endständigen OH-Gruppen können ebenfalls eingesetzt werden. Als mehrwertige Alkohole können erfindungsgemäß Xylit, Propylenglycole, Polyethylenglycole, insbesondere solche mit mittleren Molekulargewichten von 200 bis 800 g/mol, eingesetzt werden. Das mittlere Molekulargewicht kann beispielsweise mittels der DIN 55672 unter Verwendung von Polystyrol als internem Standard bestimmt werden.

Für bestimmte Anwendungsbereiche kann es im Rahmen der Erfindung vorteilhaft sein, wenn ausschließlich Sorbit oder Glycerin oder 1,2-Propylenglycol als Feuchthaltemittel eingesetzt werden. In den meisten Fällen ist dabei der Einsatz von Sorbit oder Glycerin bevorzugt. In diesem Zusammenhang kann es vorgesehen sein, dass bei alleiniger Verwendung von Sorbit in den erfindungsgemäßen Zahnreinigungsmitteln das Sorbit in einer Menge von 20 bis 60 Gew.-%, insbesondere 25 bis 50 Gew.-%, vorzugsweise 30 bis 50 Gew.-%, bezogen auf das Gesamtgewicht des Mund- und Zahnreinigungsmittels, enthalten ist. Weiterhin kann es vorgesehen sein, dass bei alleiniger Verwendung von Glycerin in den erfindungsgemäßen Zahnreinigungsmitteln das Glycerin in einer Menge von 0 bis 60 Gew.-%, insbesondere 5 bis 50 Gew.-%, vorzugsweise 10 bis 45 Gew.-%, bezogen auf das Gesamtgewicht des Mund- und Zahnreinigungsmittels, enthalten ist.

Allerdings können auch Mischungen von Sorbit, Glycerin und 1,2-Propylenglycol bevorzugt sein. Als besonders vorteilhaft hat sich in diesem Zusammenhang eine Mischung aus Sorbit und Glycerin in einem Gewichtsverhältnis von (1-1,6) : (0-1,4) erwiesen. Vorteilhaft ist darüber hinaus auch der Einsatz einer Mischung aus Glycerin, Sorbit und 1,2-Propylenglycol in einem Gewichtsverhältnis von 1 : (0,5-1) : (0,1-0,5).

Zur Unterstützung des Wirkstoffs für die orale Prophylaxe (D) können die erfindungsgemäßen Mund- und Zahnreinigungsmittel einen wundheilenden und entzündungshemmenden Stoff enthalten, welcher das entzündete, gereizte oder sensible Zahnfleisch beruhigt und dessen Heilung fördert bzw. unterstützt. In diesem Zusammenhang eignen sich beispielsweise Allantoin, Azulen, Kamillenextrakte, Tocopherol, Panthenol, Bisabolol, Salbeiextrakte sowie deren Mischungen. Besonders bevorzugt sind Mund- und Zahnreinigungsmittel, welche den wundheilenden und entzündungshemmenden Stoff in einer Menge von 0,001 bis 2,0 Gew.-%, insbesondere 0,01 bis 1,0 Gew.-%, vorzugsweise 0,05 bis 0,25 Gew.-%, bezogen auf das Gesamtgewicht des Mund- und Zahnreinigungsmittels, enthalten.

Ein Extrakt im Sinne der vorliegenden Erfindung ist ein Stoff oder Stoffgemisch, welches durch Extraktion gewonnen wurde. Die Angaben zum Gewichtsanteil des Extrakts am Gesamtgewicht erfindungsgemäßer Mund- und Zahnreinigungsmittel beziehen sich also auf die aus dem Extraktionsgut durch Extraktion erhaltenen Stoffe oder Stoffgemische, nicht jedoch auf etwaige Hilfs- oder Begleitstoffe, wie die zur Extraktion eingesetzten Lösungsmittel.

Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, dass aus einem Teil Droge höchstens 2 Teile Fluid-Extrakt gewonnen werden und *zähflüssige Extrakte bzw. Dickextrakte,* d.h. Extrakte, bei welchen ein Teil des Lösungsmittels verdampft wurde. Erfindungsgemäß geeignet sind sämtliche Extrakte, bevorzugt werden jedoch aus Kostengründen Extrakte aus den Blättern eingesetzt.

Die erfindungsgemäß verwendeten Extrakte werden durch Extraktion vorzugsweise mit organischen Lösemitteln, Wasser oder deren Mischungen, gewonnen. Geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei C₁₋₆-Alkohole, wie Ethanol und Isopropanol, und zwar sowohl als alleiniges Extraktionsmittel als auch in Mischung mit Wasser, bevorzugt. Auch solventfreie Extraktionsmethoden, einschließlich der überkritischen CO₂-Extraktion sind einsetzbar.

Die erfindungsgemäßen Mund- und Zahnreinigungsmittel können weiterhin mindestens ein Aromaöl und/oder einen Süßstoff enthalten, um die organoleptischen Eigenschaften zu verbessern. Bevorzugt wird das Aromaöl in einer Menge von 0,01 bis 5 Gew.-%, insbesondere 0,03 bis 4 Gew.-%, vorzugsweise 0,05 bis 3 Gew.-%, bevorzugt 0,1 bis 1,5 Gew.-%, bezogen auf das Gesamtgewicht des Mund- und Zahnreinigungsmittels, eingesetzt. Weiterhin kann der Süßstoff in den erfindungsgemäßen Mitteln, welche als Zahnreinigungsmittel konfektioniert sind, in einer Menge von 0,001 bis 2 Gew.-%, insbesondere 0,005 bis 1 Gew.-%, vorzugsweise 0,01 bis 0,5 Gew.-%, bezogen auf das Gesamtgewicht des Mund- und Zahnreinigungsmittels, eingesetzt werden. Sind die erfindungsgemäßen Mittel hingegen als Mundwasser bzw. Mundspüllösung konfektioniert, so kann der Süßstoff bevorzugt in einer Menge von 0,001 bis 1 Gew.-%, insbesondere 0,01 bis 0,5 Gew.-%, vorzugsweise 0,03 bis 0,3 Gew.-%, bezogen auf das Gesamtgewicht des Mund- und Zahnreinigungsmittels, eingesetzt werden.

Als Aromaöle können alle für Mund- und Zahnreinigungsmittel üblichen natürlichen und synthetischen Aromen eingesetzt werden. Natürliche Aromen können sowohl in Form der aus Drogen isolierten natürlichen ätherischen Öle als auch in Form der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten. Geeignete Süßungsmittel sind z.B. Saccharin-Natrium, Natrium-Cyclamat, Sucrose, Lactose, Meltose, Fructose, Maltose und Dextrose.

Gemäß einer weiteren Ausführungsform der vorliegenden Erfindung können die Mund- und Zahnreinigungsmittel weiterhin Konservierungsmittel in einer Menge von 0,01 bis 0,5 Gew.-%, insbesondere 0,05 bis 0,3 Gew.-%, vorzugsweise 0,1 bis 0,2 Gew.-%, bezogen auf das Gesamtgewicht des Mund- und Zahnreinigungsmittels, enthalten, um die erfindungsgemäßen Mund- und Zahnreinigungsmittel vor mikrobieller Verkeimung und daraus resultierendem Verderb zu schützen und eine lange Haltbarkeit zu gewährleisten. Hierfür können im Rahmen der Erfindung beispielsweise Ester der p-Hydroxybenzoesäure (auch als Parabene bezeichnet) sowie Sorbinsäure oder Natriumbenzoat eingesetzt werden.

Im Rahmen der vorliegenden Erfindung ist es weiterhin möglich, den erfindungsgemäßen Mund-und Zahnreinigungsmitteln Farbstoffe bzw. Pigmente zuzusetzen, um eine ästhetisch ansprechende Farbgebung zu erzielen. In den erfindungsgemäßen Mundreinigungsmitteln sind wasserlösliche Farbstoffe besonders bevorzugt. Die erfindungsgemäßen Zahnreinigungsmittel können sowohl Farbstoffe als auch Farbpigmente, wie Titandioxid, enthalten.

Zudem können die erfindungsgemäßen Mund- und Zahnreinigungsmittel auch pH-Stellmittel und Puffersubstanzen, wie Citronensäure und deren Salze oder Phosphorsäure und deren Alkalisalze zur Einstellung eines physiologisch günstigen pH-Wertes enthalten. Dadurch wird gewährleistet, dass die erfindungsgemäßen Mund- und Zahnreinigungsmittel einen ähnlichen pH-Wert wie die Mundschleimhaut aufweisen und somit besonders schleimhautverträglich sind.

Die erfindungsgemäßen Mund- und Zahnreinigungsmittel können als Mundwasser oder Mundspülung formuliert sein. Weiterhin ist es auch möglich, die erfindungsgemäßen Mund- und Zahnreinigungsmittel in Form von Mund- und Zahnpasten, flüssigen Mund- und Zahncremes sowie Mund- und Zahngelen zu formulieren.

Ein zweiter Gegenstand der vorliegenden Erfindung ist die Verwendung des erfindungsgemäßen Mund- und Zahnreinigungsmittels zur Reinigung und Pflege der Zähne und/oder der Mundhöhle. Die Reinigung der Zähne kann insbesondere mittels manuell betätigter oder elektrischer Zahnbürsten erfolgen. Im Falle elektrischer Zahnbürsten besitzen die erfindungsgemäßen Mund-und Zahnreinigungsmittel den weiteren Vorteil, dass sie bereits in geringen Mengen wirksam sind und darüber hinaus die Mechanik des elektrischen Bürstenkopfes nicht beeinträchtigen. Weiterhin kann die Reinigung der Mundhöhle insbesondere durch Spülen bzw. Ausspülen der Mundhöhle unter Verwendung des erfindungsgemäßen Mund- und Zahnreinigungsmittels erfolgen. Bezüglich der Verwendung der erfindungsgemäßen Mund- und Zahnreinigungsmittel gilt mutatis mutandis das zu den erfindungsgemäßen Mund- und Zahnreinigungsmitteln gesagt.

Ein dritter Gegenstand der vorliegenden Erfindung ist die Verwendung des erfindungsgemäßen Mund- und Zahnreinigungsmittels zur Erhöhung der Schaummenge und/oder zur Verbesserung der Schaumkonsistenz. Die Erhöhung der Schaummenge und/oder die Verbesserung der Schaumkonsistenz wird erfindungsgemäß durch die spezifische und zielgerichtete Kombination der Tenside (A), (B) und (C) erreicht und kann durch den Einsatz der Tenside (A), (B) und (C) in ganz speziellen Gewichtsverhältnissen und Mengenbereichen weitergehend gesteigert werden. Zudem kann durch diese Kombination die Menge an hautreizenden Alkyl(ether)sulfaten gegenüber dem Stand der Technik signifikant reduziert werden, so dass ein besonders schleimhautverträgliches Mund- und Zahnreinigungsmittel resultiert, welches sich insbesondere bei entzündetem, gereiztem und sensiblem Zahnfleisch eignet. Bezüglich der Verwendung der erfindungsgemäßen Mund- und Zahnreinigungsmittel gilt mutatis mutandis das zu den erfindungsgemäßen Mund- und Zahnreinigungsmitteln gesagt.

Ein vierter Gegenstand der vorliegenden Erfindung ist ein Verfahren zur Reinigung und Pflege der Zähne und/oder der Mundhöhle, wobei das erfindungsgemäße Mund- und Zahnreinigungsmittel auf eine Zahnbürste aufgetragen und mit dieser die Zähne geputzt werden und/oder wobei die Mundhöhle mit dem erfindungsgemäßen Mund- und Zahnreinigungsmittel gespült und/oder ausgespült wird. Aufgrund der hohen Schaummenge sowie der guten Schaumstabilität resultiert das erfindungsgemäße Verfahren in einer äußerst schleimhautverträglichen und hervorragenden Reinigung und Pflege der Zähne und der Mundhöhle. Bezüglich des Verfahrens gilt mutatis mutandis das zu den erfindungsgemäßen Mund- und Zahnreinigungsmitteln gesagt.

Die folgenden Beispiele sind rein zur Erläuterung:

### Beispiele

Es wurden die folgende Formulierungen hergestellt, wobei alle Zahlenwerte in den nachfolgenden Tabellen 1 und 2 - sofern nicht anders angegeben - der Menge des jeweiligen Rohstoffs in Gewichtsprozent entsprechen:

**Tabelle 1: erfindungsgemäße Zahnpastaformulierungen**

| | **Formulierungen** | | | | | | | | |
|---|---|---|---|---|---|---|---|---|---|
| | **1*** | **2*** | **3*** | **4*** | **5*** | **6*** | **7*** | **8*** | **9** |
| **Sorbit** | | | 50 | 50 | 65 | 65 | 60 | 60 | 60 |
| **(70% AS)** | | | (35) | (35) | (46) | (46) | (42) | (42) | (42) |
| **Glycerin** | 35 | 35 | | | 12 | 12 | 7,0 | 7,0 | 7,0 |
| **Hydrated silica** | 4,0 | 4,0 | 16 | 16 | 12 | 12 | 8,0 | 8,0 | 8,0 |
| **Natriumfluorid** | 0,12 | 0,12 | 0,16 | 0,16 | 0,20 | 0,20 | 0,28 | 0,28 | 0,28 |
| **Saccharin-Natrium** | 0,14 | 0,14 | 0,22 | 0,22 | 0,30 | 0,30 | 0,42 | 0,42 | 0,42 |
| **Aroma** | 0,25 | 0,25 | 0,55 | 0,55 | 0,85 | 0,85 | 1,25 | 1,25 | 1,25 |
| **Ethanol** | | | 1,8 | 1,8 | 3,0 | 3,0 | 4,2 | 4,2 | 4,2 |
| **Keltrol** | 0,20 | 0,20 | 0,47 | 0,47 | 0,65 | 0,65 | 0,83 | 0,83 | 0,83 |
| **Texapon LS 35** | 0,30 | 0,30 | 0,50 | 0,50 | 0,85 | 0,85 | 1,1 | 1,1 | 1,1 |
| **Tego Betain** F **50** | 0,42 | 0,90 | 0,70 | 1,5 | 1,2 | 2,5 | 1,5 | 3,3 | |
| **Plantacare® 818UP** | 0,42 | 0,15 | 0,70 | 0,25 | 1,2 | 0,40 | 1,5 | 0,55 | |
| **Gantrez ES 425** | | | 0,4 | 0,4 | 0,8 | 0,8 | 1,5 | 1,5 | 1,5 |
| **Wasser** | ad 100 | ad 100 | ad 100 | ad 100 | ad 100 | ad 100 | ad 100 | ad 100 | ad 100 |

| | | | | | | | | | |
|---|---|---|---|---|---|---|---|---|---|
| *erfindungsgemäße Formulierungen | | | | | | | | | |

**Tabelle 2: erfindungsgemäße Mundwasserformulierungen**

| | **Formulierungen** | | | | | | | | |
|---|---|---|---|---|---|---|---|---|---|
| | **10*** | **11*** | **12*** | **13*** | **14*** | **15*** | **16*** | **17*** | **18** |
| **Sorbit** | 1,0 | 1,0 | 2,0 | 2,0 | 3,0 | 3,0 | 5,0 | 5,0 | 5,0 |
| **(70% AS)** | (0,7) | (0,7) | (1,4) | (1,4) | (2,1) | (2,1) | (3,5) | (3,5) | (3,5) |
| **Emulgin HRE 60** | 0,10 | 0,10 | 0,30 | 0,30 | 0,60 | 0,60 | 1,0 | 1,0 | 1,0 |
| **Natriumfluorid** | 0,11 | 0,11 | 0,11 | 0,11 | 0,11 | 0,11 | 0,11 | 0,11 | 0,11 |
| **Trinatriumcitrat Dihydrat** | 0,10 | 0,10 | 0,20 | 0,20 | 0,40 | 0,40 | 0,60 | 0,60 | 0,60 |
| **Citronensäure** | 0,001 0 | 0,001 0 | 0,002 5 | 0,002 5 | 0,005 0 | 0,005 0 | 0,01 | 0,01 | 0,01 |
| **Saccharin-Natrium** | 0,030 | 0,030 | 0,080 | 0,080 | 0,15 | 0,15 | 0,30 | 0,30 | 0,30 |
| **Ethanol** | 5 | 5 | 10 | 10 | 20 | 20 | 30 | 30 | 30 |
| **Aroma** | 0,10 | 0,10 | 0,30 | 0,30 | 0,60 | 0,60 | 1,0 | 1,0 | 1,0 |
| **Wirkstoff (D)** | 0,010 | 0,010 | 0,030 | 0,030 | 0,060 | 0,060 | 0,10 | 0,10 | 0,10 |
| **Texapon LS 35** | 0,30 | 0,30 | 0,50 | 0,50 | 0,85 | 0,85 | 1,1 | 1,1 | 1,1 |
| **Tego Betain F 50** | 0,42 | 0,90 | 0,70 | 1,5 | 1,2 | 2,5 | 1,5 | 3,3 | |
| **Plantacare® 818UP** | 0,42 | 0,15 | 0,70 | 0,25 | 1,2 | 0,40 | 1,5 | 0,55 | |
| **Gantrez ES 425** | 0,10 | 0,10 | 0,20 | 0,20 | 0,35 | 0,35 | 0,50 | 0,50 | 0,50 |
| **Wasser** | ad 100 | ad 100 | ad 100 | ad 100 | ad 100 | ad 100 | ad 100 | ad 100 | ad 100 |

| | | | | | | | | | |
|---|---|---|---|---|---|---|---|---|---|
| *erfindungsgemäße Formulierungen | | | | | | | | | |

Es wurden die folgenden Handelsprodukte eingesetzt:
- Keltrol:: INCI-Bezeichnung: Xanthan Gum; CP Kelco
- Texapon LS 35:: INCI-Bezeichnung: Sodium lauryl sulfate; BASF
- Plantacare^{®} 818UP:: INCI-Bezeichnung: Coco-Glucoside, ca. 50 % Aktivsubstanz; Cognis
- Tego Betain F 50:: INCI-Bezeichnung: Cocamidopropyl betain, Evonik Goldschmidt
- Gantrez ES 425:: INCI-Bezeichnung: Butyl Ester of PVM/MA Copolymer, ISP
- Emulgin HRE 60:: INCI-Bezeichnung: PEG-60 Hydrogenated Castor Oil, BASF

Die Herstellung der Zahnpastaformulierungen 1 bis 9 erfolgt nach folgender allgemeiner Vorschrift: Das Glycerin und das Sorbit werden vorgelegt. Anschließend wird das Bindemittel Keltrol in kleinen Portionen unter Rühren hinzugegeben. Nacheinander werden in diese Mischung vollentsalztes Wasser, Saccharin-Natrium, Natriumfluorid und gegebenenfalls Gantrez ES 425 eingerührt. Zum Quellen des Bindemittels wird die Mischung etwa 15 min gerührt. Wenn dieser Teilansatz homogen ist, werden unter ständigem Rühren Hydrated silica und die Tenside (erfindungsgemäße Formulierungen) bzw. das Tensid (nichterfindungsgemäße Formulierung) hinzugefügt. Das Aromaöl wird mit Ethanol versetzt und untergerührt. Das resultierende Gemisch wird solange homogenisiert, bis eine vollständige Durchmischung aller Komponenten und eine glatte Struktur erreicht sind.

Die Herstellung der Mundwasserformulierungen 10 bis 18 erfolgt nach folgender allgemeiner Vorschrift:

Das Sorbit wird mit Ethanol, Natriumfluorid, Wirkstoff (D), Saccharin-Natrium, Trinatriumcitrat Dihydrat, einem Teil des vollentsalzten Wassers sowie Gantrez ES 425 versetzt und etwa 15 min gerührt. Wenn diese Mischung homogen ist, werden die Tenside (erfindungsgemäße Formulierungen) bzw. das Tensid (nichterfindungsgemäße Formulierung) hinzugefügt. Das Aromaöl wird mit Emulgin HRE 60 vermischt und hinzugegeben. Nach Einstellung des pH-Wertes mit Citronensäure wird mit dem restlichen vollentsalzten Wasser aufgefüllt und solange gerührt, bis eine homogene Lösung erhalten wird.

### Messung der Schaummenge:

Die Messung der Schaummenge sowie der Schaumstabilität erfolgt mittels eines SITA Foam Testers R-2000 (Firma SITA Messtechnik GmbH, Deutschland) sowie der Software "SITA-foam" (Version 1.0). Pro Probe werden jeweils 3 Messungen durchgeführt.

Für die Messung der Schaummenge werden die folgenden Parameter verwendet:

| | |
|---|---|
| Probenkonzentration: | 10%ige Lösung der Zahnpasta- oder Mundwasserformulierung in vollentsalztem Wasser |
| Temperatur: | 27 ± 2 °C |
| Rotordrehzahl: | 1200 U/min |
| Rührdauer: | 10 s |
| Probenmenge: | 250 ml |

Die Messung der Schaumstabilität erfolgt unmittelbar im Anschluss an die Messung der Schaummenge, wobei die folgenden Parameter verwendet werden:

| | |
|---|---|
| Probenkonzentration: | 10%ige Lösung der Zahnpasta- oder Mundwasserformulierung in vollentsalztem Wasser |
| Temperatur: | 27 ± 2 °C |
| Messintervall: | 10 s |
| Gesamtdauer Messung: | 3 min |

### Ergebnisse:

Für die erfindungsgemäßen Formulierungen 1 bis 8 und 10 bis 17 mit der spezifischen Kombination der Tenside (A), (B) und (C) in einem definierten Gewichtsverhältnis wird eine signifikant gesteigerte Schaummenge im Vergleich zu den nichterfindungsgemäßen Formulierungen 9 und 18 erhalten. Für die erfindungsgemäßen Formulierungen 5 und 6 sowie 14 und 15 wird sogar eine mehr als doppelt so große Schaummenge erhalten wie für die nichterfindungsgemäßen Formulierungen 9 und 18. Somit kann die Schaummenge durch die gezielte Kombination der Tenside (A), (B) und (C) in speziellen Gewichtsverhältnissen und Mengenbereichen weitergehend gesteigert werden.

Aufgrund der hohen Schaummenge erfolgt eine gute Verteilung der Inhaltsstoffe, weshalb die erfindungsgemäßen Formulierungen 1 bis 8 und 10 bis 17 eine hervorragende Reinigungsleistung aufweisen.

Weiterhin sind die erfindungsgemäßen Formulierungen 1 bis 8 und 10 bis 17 aufgrund des geringen Gehalts an hautreizenden Alkylsulfaten besonders schleimhautverträglich und eignen sich somit insbesondere für die Verwendung bei entzündetem, gereiztem und sensiblem Zahnfleisch.

Die erfindungsgemäßen Formulierungen 1 bis 8 und 10 bis 17 weisen weiterhin eine hervorragende Schaumstabilität während der Zahnreinigung auf, so dass eine Verflüssigung und ein Herauslaufen des Schaums aus dem Mund nicht beobachtet werden.

Schließlich weisen die erfindungsgemäßen Formulierungen 1 bis 8 und 10 bis 17 ebenfalls eine hervorragende Schaumqualität, insbesondere einen feinporigen und cremigen Schaum auf und besitzen folglich hervorragende sensorische Eigenschaften. Weiterhin schäumen die erfindungsgemäßen Formulierungen 1 bis 8 und 10 bis 17 äußerst schnell auf, so dass die vom Verbraucher gewünschte hohe Schaummenge bereits nach sehr kurzer Zeit zur Verfügung steht.

## Patentansprüche

1. Mund- und Zahnreinigungsmittel, enthaltend
a) 0,1 bis 3 Gew.-%, bezogen auf das Gesamtgewicht des Mund- und Zahnreinigungsmittels, mindestens eines Alkyl(ether)sulfats (A) der allgemeinen Formel (I)
R¹-(O-CH₂-CH₂)ₓ-O-SO₃- X⁺ (I),
in der R¹ für eine lineare oder verzweigte, gesättigte oder ungesättigte Alkylgruppe mit 8 bis 30 Kohlenstoffatomen, x für die Zahl 0 oder für Zahlen von 1 bis 12 und X⁺ für ein Alkalimetall- oder ein Ammoniumion steht,
b) 0,1 bis 4 Gew.-%, bezogen auf das Gesamtgewicht des Mund- und Zahnreinigungsmittels, mindestens eines amphoteren Tensids (B) der allgemeinen Formel (II) 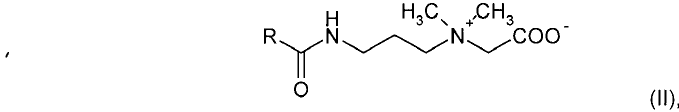 in der R für eine lineare oder verzweigte, gesättigte oder ein- oder mehrfach ungesättigte Alkyl- oder Alkenylgruppe mit 8 bis 24 Kohlenstoffatomen steht,
c) 0,1 bis 2 Gew.-%, bezogen auf das Gesamtgewicht des Mund- und Zahnreinigungsmittels, mindestens eines nichtionischen Tensids (C) der allgemeinen Formel (III)
R²O-[G]ₚ (III),
in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, und
d) 0,1 bis 1 Gew.-%, bezogen auf das Gesamtgewicht des Mund- und Zahnreinigungsmittels, mindestens eines Wirkstoffs für die orale Prophylaxe (D), ausgewählt aus der Gruppe der Fluoride, der antibakteriellen Wirkstoffe und der Phosphate,
wobei das Gewichtsverhältnis derTenside (A) : (B) : (C) 0,7 : 1,8 : 1,8 bis 1 : 3,5 : 0,5 beträgt.

2. Mund- und Zahnreinigungsmittel nach Anspruch 1, **dadurch gekennzeichnet, dass** das mindestens eine Alkyl(ether)sulfat (A) der allgemeinen Formel (I) ausgewählt ist aus der Gruppe der geradkettigen oder verzweigten Alkyl(ether)sulfate mit einem Alkylrest mit 8 bis 18, insbesondere mit 10 bis 16 Kohlenstoffatomen und 0 oder 1 bis 6 Ethylenoxideinheiten, bevorzugt Natriumlaurylsulfat und/oder Natriumlaurethsulfat.

3. Mund- und Zahnreinigungsmittel nach einem der Ansprüche 1 oder 2, **dadurch gekennzeichnet, dass** das mindestens eine Alkyl(ether)sulfat (A) der allgemeinen Formel (I) in einer Menge von 0,1 bis 2,5 Gew.-%, insbesondere 0,3 bis 2 Gew.-%, vorzugsweise 0,4 bis 1,5 Gew.-%, bevorzugt 0,5 bis 1,3 Gew.-%, besonders bevorzugt 0,6 bis 1 Gew.-%, am meisten bevorzugt 0,7 bis 0,9 Gew.-%, bezogen auf das Gesamtgewicht des Mund- und Zahnreinigungsmittels, enthalten ist.

4. Mund- und Zahnreinigungsmittel nach einem der vorangehenden Ansprüche, **dadurch gekennzeichnet, dass** das mindestens eine amphotere Tensid (B) der allgemeinen Formel (II) ausgewählt ist aus der Gruppe der C₈₋₁₈-Alkylamido(C₁₋₄)-alkylbetaine, insbesondere C₈₋₁₂-Alkylamido(C₁₋₄)-alkylbetaine.

5. Mund- und Zahnreinigungsmittel nach einem der vorangehenden Ansprüche, **dadurch gekennzeichnet, dass** das mindestens eine amphotere Tensid (B) der allgemeinen Formel (II) in einer Menge von 0,1 bis 3,7 Gew.-%, insbesondere 0,5 bis 3,5 Gew.-%, vorzugsweise 1 bis 3,3 Gew.-%, bevorzugt 1,5 bis 3 Gew.-%, besonders bevorzugt 2,2 bis 2,8 Gew.-%, bezogen auf das Gesamtgewicht des Mund- und Zahnreinigungsmittels, enthalten ist.

6. Mund- und Zahnreinigungsmittel nach einem der vorangehenden Ansprüche, **dadurch gekennzeichnet, dass** das mindestens eine nichtionische Tensid (C) der allgemeinen Formel (III) ausgewählt ist aus der Gruppe der Alkyloligoglucoside mit 8 bis 16 Kohlenstoffatomen in der Alkylgruppe und deren Mischungen.

7. Mund- und Zahnreinigungsmittel nach einem der vorangehenden Ansprüche, **dadurch gekennzeichnet, dass** das mindestens eine nichtionische Tensid (C) der allgemeinen Formel (III) in einer Menge von 0,1 bis 1,5 Gew.-%, insbesondere 0,1 bis 1 Gew.-%, vorzugsweise 0,1 bis 0,8 Gew.-%, bevorzugt 0,2 bis 0,6 Gew.-%, mehr bevorzugt 0,3 bis 0,5 Gew.-%, bezogen auf das Gesamtgewicht des Mund- und Zahnreinigungsmittels, enthalten ist.

8. Mund- und Zahnreinigungsmittel nach einem der vorangehenden Ansprüche, **dadurch gekennzeichnet, dass** der mindestens eine Wirkstoff für die orale Prophylaxe (D) ausgewählt ist aus der Gruppe der (i) Natriumfluoride; (ii) Dinatriummonofluorphosphate; (iii) Kaliumfluoride; (iv) Zinn(II)-fluoride; (v) Aminfluoride sowie (vi) deren Mischungen, insbesondere Natriumfluorid; und/oder dass der mindestens eine Wirkstoff für die orale Prophylaxe (D) ausgewählt ist aus Cetylpyridiniumchlorid, Chlorhexidin, Hexetidin, Domiphenchlorid, Triclosan, Benzoaten sowie deren Mischungen, insbesondere Cetylpyridiniumchlorid; und/oder dass der mindestens eine Wirkstoff für die orale Prophylaxe (D) ausgewählt ist der Gruppe der (i) Ethylendiamintetraessigsäure und deren Natriumsalzen; (ii) Pyrophosphatsalze, insbesondere wasserlösliche Dialkali- oder Tetraalkalimetallpyrophosphatsalze; (iii) Polyphosphatsalze, insbesondere Alkalimetalltripolyphosphate, Natriumtripolyphosphate und Kaliumtripolyphosphate; sowie (iv) deren Mischungen.

9. Mund- und Zahnreinigungsmittel nach einem der vorangehenden Ansprüche, **dadurch gekennzeichnet, dass** der mindestens eine Wirkstoff für die orale Prophylaxe (D) in einer Menge von 0,1 bis 0,8 Gew.-%, insbesondere 0,1 bis 0,7 Gew.-%, vorzugsweise 0,1 bis 0,6 Gew.-%, bevorzugt 0,1 bis 0,5 Gew.-%, besonders bevorzugt 0,1 bis 0,3 Gew.-%, bezogen auf das Gesamtgewicht des Mund- und Zahnreinigungsmittels, enthalten ist.

10. Mund- und Zahnreinigungsmittel nach einem der vorangehenden Ansprüche, **dadurch gekennzeichnet, dass** das Mund- und Zahnreinigungsmittel teilchenförmige Polierkomponenten, ausgewählt aus der Gruppe der Fällungs- und Gelkieselsäuren, Aluminiumhydroxide, Aluminiumoxide, Natriumaluminiumsilikate, organischen Polymere, Hydroxylapatite, Natriumaluminiumsilicate sowie deren Mischungen, insbesondere Fällungs- und Gelkieselsäuren, enthält, wobei die teilchenförmigen Polierkomponenten einen mittleren Teilchendurchmesser D50 von kleiner 15 µm aufweisen, und/oder wobei die teilchenförmigen Polierkomponenten in einer Menge von 1 bis 40 Gew.-%, insbesondere 2 bis 35 Gew.-%, vorzugsweise 3 bis 30 Gew.-%, bevorzugt 4 bis 25 Gew.-%, besonders bevorzugt 5 bis 20 Gew.-%, bezogen auf das Gesamtgewicht des Mund- und Zahnreinigungsmittels, enthalten sind.

11. Mund- und Zahnreinigungsmittel nach einem der vorangehenden Ansprüche, **dadurch gekennzeichnet, dass** das Mund- und Zahnreinigungsmittel mindestens einen weiteren Inhaltsstoff, ausgewählt aus der Gruppe der Lösungsvermittler, Bindemittel, Feuchthaltemittel und Konsistenzgeber, Aromaöle, Süßstoffe, Konservierungsmittel, Lösungsmittel, Farbstoffe, pH-Stellmittel und Puffersubstanzen, enthält.

12. Verwendung eines Mund- und Zahnreinigungsmittels nach einem der Ansprüche 1 bis 11 zur Reinigung und Pflege der Zähne und/oder der Mundhöhle.

13. Verwendung eines Mund- und Zahnreinigungsmittels nach einem der Ansprüche 1 bis 11 zur Erhöhung der Schaummenge und/oder zur Verbesserung der Schaumkonsistenz.

## Claims

1. An oral and dental cleaning agent, containing
a) 0.1 to 3 wt. %, based on the total weight of the oral and dental cleaning agent, of at least one alkyl(ether)sulfate (A) of the general formula (I)
R¹-(O-CH₂-CH₂)ₓ-O-SO₃ X⁺ (I),
in which R¹ represents a linear or branched, saturated or unsaturated alkyl group having from 8 to 30 carbon atoms, x represents the number 0 or numbers 1 to 12, and X⁺ represents an alkali metal ion or an ammonium ion,
b) 0.1 to 4 wt. %, based on the total weight of the oral and dental cleaning agent, of at least one amphoteric surfactant (B) of the general formula (II) 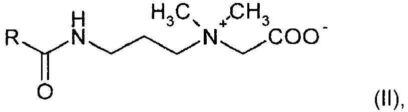 in which R represents a linear or branched, saturated or mono- or polyunsaturated alkyl or alkenyl group having 8 to 24 carbon atoms,
c) 0.1 to 2 wt. %, based on the total weight of the oral and dental cleaning agent, of at least one non-ionic surfactant (C) of the general formula (III)
R²O-[G]ₚ (III),
in which R² represents an alkyl and/or alkenyl group having 4 to 22 carbon atoms, G represents a sugar functional group having 5 or 6 carbon atoms, and p represents numbers from 1 to 10, and
d) 0.1 to 1 wt. %, based on the total weight of the oral and dental cleaning agent, of at least one active ingredient for oral prophylaxis (D), selected from the group of fluorides, antibacterial active ingredients and phosphates, wherein the weight ratio of the surfactants is (A) : (B) : (C) 0.7 : 1.8 : 1.8 to 1 : 3.5 : 0.5.

2. The oral and dental cleaning agent according to claim 1, **characterized in that** the at least one alkyl (ether) sulfate (A) of the general formula (I) is selected from the group of straight-chain or branched alkyl (ether) sulfates that have one alkyl functional group having 8 to 18, in particular 10 to 16 carbon atoms and 0 or 1 to 6 ethylene oxide units, preferably sodium lauryl sulfate and/or sodium laureth sulfate.

3. The oral and dental cleaning agent according to claim 1 or 2, **characterized in that** it contains the at least one alkyl (ether) sulfate (A) of the general formula (I) in an amount of 0.1 to 2.5 wt. %, in particular 0.3 to 2 wt. %, preferably 0.4 to 1.5 wt. %, preferably 0.5 to 1.3 wt. %, particularly preferably 0.6 to 1 wt. %, most preferably 0.7 to 0.9 wt. %, based on the total weight of the oral and dental cleaning agent.

4. The oral and dental cleaning agent according to one of the preceding claims, **characterized in that** the at least one amphoteric surfactant (B) of the general formula (II) is selected from the group of C₈-C₁₈ alkylamido(C₁-₄)alkyl betaines, in particular C₈-₁₂ alkylamido(C₁-₄)alkyl betaines.

5. The oral and dental cleaning agent according to one of the preceding claims, **characterized in that** it contains the at least one amphoteric surfactant (B) of the general formula (II) in an amount of 0.1 to 3.7 wt. %, in particular 0.5 to 3.5 wt. %, preferably 1 to 3.3 wt. %, preferably 1.5 to 3 wt. %, particularly preferably 2.2 to 2.8 wt. %, based on the total weight of the oral and dental cleaning agent.

6. The oral and dental cleaning agent according to one of the preceding claims, **characterized in that** the at least one non-ionic surfactant (C) of the general formula (III) is selected from the group of alkyl oligoglucosides having 8 to 16 carbon atoms in the alkyl group and mixtures thereof.

7. The oral and dental cleaning agent according to one of the preceding claims, **characterized in that** it contains the at least one non-ionic surfactant (C) of the general formula (III) in an amount of 0.1 to 1.5 wt. %, in particular 0.1 to 1 wt. %, preferably 0.1 to 0.8 wt. %, preferably 0.2 to 0.6 wt. %, more preferably 0.3 to 0.5 wt. %, based on the total weight of the oral and dental cleaning agent.

8. The oral and dental cleaning agent according to one of the preceding claims, **characterized in that** the at least one active ingredient for oral prophylaxis (D) is selected from the group of (i) sodium fluorides; (ii) disodium monofluorophosphates; (iii) potassium fluorides; (iv) tin(II) fluorides; (v) amine fluorides and (vi) mixtures thereof, in particular sodium fluoride; and/or
**in that** the at least one active ingredient for oral prophylaxis (D) is selected from cetylpyridinium chloride, chlorohexidine, hexetidine, domiphen chloride, triclosan, benzoates and mixtures thereof, in particular cetylpyridinium chloride;
and/or **in that** the at least one active ingredient for oral prophylaxis (D) is selected from the group of (i) ethylendiaminetetraacetic acid and sodium salts thereof; (ii) pyrophosphate salts, in particular water-soluble dialkali or tetraalkali metal pyrophosphate salts; (iii) polyphosphate salts, in particular alkali metal tripolyphosphates, sodium tripolyphosphates and potassium polyphosphates, and (iv) mixtures thereof.

9. The oral and dental cleaning agent according to one of the preceding claims, **characterized in that** it contains the at least one active ingredient for oral prophylaxis (D) in an amount of 0.1 to 0.8 wt. %, in particular 0.1 to 0.7 wt. %, preferably 0.1 to 0.6 wt. %, preferably 0.1 to 0.5 wt. %, particularly preferably 0.1 to 0.3 wt. %, based on the total weight of the oral and dental cleaning agent.

10. The oral and dental cleaning agent according to one of the preceding claims, **characterized in that** the oral and dental cleaning agent contains particulate polishing components, selected from the group of precipitated silica and silica gels, aluminum hydroxides, aluminum oxides, sodium aluminum silicates, organic polymers, hydroxylapatites, sodium aluminum silicates and mixtures thereof, in particular precipitated silica and silica gels, the particulate polishing components having an average particle diameter D50 of less than 15 µm, and/or the oral and dental cleaning agent containing the particulate polishing components in an amount of 1 to 40 wt. %, in particular 2 to 35 wt. %, preferably 3 to 30 wt. %, preferably 4 to 25 wt. %, particularly preferably 5 to 20 wt. %, based on the total weight of the oral and dental cleaning agent.

11. The oral and dental cleaning agent according to one of the preceding claims, **characterized in that** the oral and dental cleaning agent contains at least one additional ingredient, selected from the group of solutizers, binders, humectants and bodying agents, aroma oils, sweeteners, preservatives, solvents, colorants, pH regulators and buffer substances.

12. The use of an oral and dental cleaning agent according to one of claims 1 to 11 for cleaning and caring for the teeth and/or the buccal cavity.

13. The use of an oral and dental cleaning agent according to one of claims 1 to 11 for increasing the amount of foam and/or to improve the foam consistency.

## Revendications

1. Agent de nettoyage bucco-dentaire contenant
a) 0,1 à 3% en poids, par rapport au poids total de l'agent de nettoyage bucco-dentaire, d'au moins un alkyl(éther)sulfate (A) de la formule générale (I)
R¹-(O-CH₂-CH₂)ₓ-O-SO₃⁻X⁺ (I)
dans laquelle R¹ représente un groupe alkyle linéaire ou ramifié, saturé ou insaturé, ayant 8 à 30 atomes de carbone, x représente le nombre 0 ou des nombres allant de 1 à 12 et X⁺ représente un ion métallique alcalin ou un ion ammonium,
b) 0,1 à 4% en poids, par rapport au poids total de l'agent de nettoyage bucco-dentaire, d'au moins un tensioactif amphotère (B) de la formule générale (II) 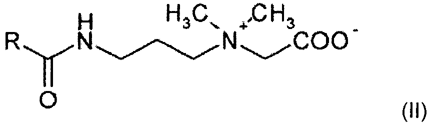 dans laquelle R représente un groupe alkyle ou alcényle linéaire ou ramifié, saturé ou monoinsaturé ou polyinsaturé ayant de 8 à 24 atomes de carbone:,
c) 0,1 à 2% en poids, par rapport au poids total de l'agent de nettoyage bucco-dentaires, d'au moins un tensioactif non ionique (C) de la formule générale (III)
R²O-[G]ₚ (III)
dans laquelle R² représente un radical alkyle et/ou alcényle ayant 4 à 22 atomes de carbone, G représente un radical sucre ayant 5 ou 6 atomes de carbone et p représente des nombres de 1 à 10, et
d) 0,1 à 1% en poids, par rapport au poids total de l'agent de nettoyage bucco-dentaire d'au moins une substance active pour la prophylaxie orale (D), choisi dans le groupe des fluorures, des agents antibactériens et des phosphates, le rapport en poids des tensioactifs (A):(B):(C) étant 0,7:1,8:1,8 à 1:3,5:0,5.

2. Agent de nettoyage bucco-dentaire selon la revendication 1, **caractérisé en ce que** l'au moins un l'alkyl(éther)sulfate (A) de la formule générale (I) est choisi dans le groupe des alkyl(éther)sulfates, linéaire ou ramifié, comportant un radical alkyle ayant 8 à 18, en particulier 10 à 16, atomes de carbone et 0 ou 1 à 6 unités d'oxyde d'éthylène, , de préférence de laurylsulfate de sodium et/ou de laureth-sulfate de sodium.

3. Agent de nettoyage bucco-dentaire selon l'une des revendications 1 ou 2, **caractérisé en ce que** l'au moins un alkyl(éther)sulfate (A) de la formule générale (I) dans une quantité de 0,1 et 2,5% en poids, en particulier de 0,3 à 2% en poids, avantageusement de 0,4 à 1,5% en poids, de préférence 0,5 à 1,3% en poids, de manière particulièrement préférée de 0,6 à 1 % en poids, le plus préférablement de 0,7 à 0,9% en poids, sur la base du poids total de l'agent de nettoyage bucco-dentaire.

4. Agent de nettoyage bucco-dentaire selon l'une des revendications précédentes, **caractérisé en ce que** l'au moins un tensioactif amphotère (B) de la formule générale (II) est choisi dans le groupe des C₈₋₁₈-alkylamido(C₁₋₄)alkylbétaine, en particulier C₈₋₁₂-alkylamido(C₁₋₄)-alkylbétaine.

5. Agent de nettoyage bucco-dentaire selon l'une des revendications précédentes, **caractérisé en ce que** l'au moins un tensio-actif amphotère (B) de la formule générale (II) est contenu dans une quantité de 0,1 à 3,7% en poids, en particulier de 0,5 à 3,5% en poids, avantageusement de 1 à 3,3% en poids, de préférence de 1,5 et 3% en poids, de façon particulièrement préférée de 2,2 à 2,8% en poids, par rapport au poids total de l'agent de nettoyage bucco-dentaire.

6. Agent de nettoyage bucco-dentaire selon l'une des revendications précédentes, **caractérisé en ce que** l'au moins un tensioactif non ionique (C) de la formule générale (III) est choisi dans le groupe des alkyloligoglucosides ayant 8 à 16 atomes de carbone dans le groupe alkyle et leurs mélanges.

7. Agent de nettoyage bucco-dentaire selon l'une des revendications précédentes, **caractérisé en ce que** l'au moins un tensioactif non ionique (C) de la formule générale (III) est contenu dans une quantité de 0,1 à 1,5% en poids, en particulier 0,1 à 1% en poids, avantageusement de 0,8 à 0,1% en poids, de préférence de 0,2 à 0,6% en poids, plus préférablement de 0,3 à 0,5% en poids, par rapport au poids total l'agent de nettoyage bucco-dentaire.

8. Agent de nettoyage bucco-dentaire selon l'une des revendications précédentes, **caractérisé en ce que** l'au moins une substance active pour la prophylaxie orale (D) est choisie dans le groupe (i) ds fluorure de sodium ; (ii) monofluorophosphate disodique ; (iii) du fluorure de potassium ; (iv) du fluorure d'étain(II) ; (v) des fluorures d'amines et (vi) leurs mélanges, en particulier le fluorure de sodium; et/ou **en ce que**
au moins une substance active pour la prophylaxie orale (D) est choisie parmi le chlorure de cétylpyridinium, la chlorhexidine, l'hexétidine, le chlorure de domiphène, le triclosan, les benzoates et leurs mélanges, en particulier le chlorure de cétylpyridinium ; et/ou **en ce que** l'au moins une substance active pour la prophylaxie orale (D) est choisie dans le groupe (i) de l'acide éthylènediaminetétracétique et ses sels de sodium ; (ii) les sels de pyrophosphate, en particulier les sels de pyrophosphates de métaux dialcalins ou tétraalcalin soluble dans l'eau ; (iii) les sels de polyphosphate, en particulier les tripolyphosphates métaux alcalins, le tripolyphosphate de sodium et de potassium ; et (iv) leurs mélanges.

9. Agent de nettoyage bucco-dentaire selon l'une des revendications précédentes, **caractérisé en ce que** l'au moins une substance active pour la prophylaxie orale (D) est contenue dans une quantité de 0,1 à 0,8% en poids, en particulier de 0,1 à 0,7% en poids, avantageusement de 0,1 à 0,6% en poids, de préférence de 0,1 à 0,5% en poids, de façon particulièrement préférée de 0,1 à 0,3% en poids, par rapport au poids total de l'agent de nettoyage bucco-dentaire.

10. Agent de nettoyage bucco-dentaire selon l'une des revendications précédentes, **caractérisé en ce que** l'agent de nettoyage bucco-dentaire contient des composants de polissage particulaires choisis dans le groupe des acides siliciques en gel et précipités, des hydroxydes d'aluminium, des oxydes d'aluminium, des silicates d'aluminium et de sodium, des polymères organiques, de l'hydroxylapatite, des silicates d'aluminium et de sodium ainsi que de leurs mélanges, en particulier les acides silices en gel et précipités, les composants de polissage particulaires ayant un diamètre de particule moyen D50 inférieur à 15 µm et/ou les composants de polissage particulaires étant contenus dans une quantité de 1 à 40% en poids, en particulier de 2 à 35% en poids, avantageusement de 3 à 30% en poids, de préférence de 4 à 25% en poids, de façon particulièrement préférée de 5 à 20% en poids, par rapport au poids total de l'agent de nettoyage bucco-dentaire.

11. Agent de nettoyage bucco-dentaire selon l'une des revendications précédentes, **caractérisé:**
**en ce que** l'agent de nettoyage bucco-dentaire contient au moins un autre ingrédient choisi dans le groupe des agents de solubilisation, des liants, des humecteurs et des régulateurs de consistance, des huiles aromatisantes, des édulcorants, des conservateurs, des solvants, des colorants, des agents d'ajustement de pH et des substances tampons.

12. Utilisation d'un agent de nettoyage bucco-dentaire selon l'une des revendications 1 à 11 pour le nettoyage et le soin des dents et/ou de la cavité buccale.

13. Utilisation d'un agent de nettoyage bucco-dentaire selon l'une des revendications 1 à 11 pour augmenter la quantité de mousse et/ou pour améliorer la consistance de la mousse.
